Europäisches Patentamt

European Patent Office

Office européen des brevets

(11) Publication number: **0 255 654**
**A1**

# (12) EUROPEAN PATENT APPLICATION

(21) Application number: 87110589.6

(22) Date of filing: 22.07.87

(51) Int. Cl.4: **D21H 5/26** , A61F 13/18 , A47L 13/16

(30) Priority: 06.08.86 IT 1252986

(43) Date of publication of application:
10.02.88 Bulletin 88/06

(84) Designated Contracting States:
AT BE CH DE ES FR GB GR IT LI LU NL SE

(71) Applicant: **MIRA LANZA S.p.a.**
**Via Rivarolo 14**
**I-16161 Genova(IT)**

(72) Inventor: **Nistri, Ugo**
**Via Coriolano Bozzo 4**
**I-16030 Pieve Ligure(IT)**
Inventor: **Colombo, Paolo**
**Via Avogadro 12**
**I-21047 Saronno(IT)**
Inventor: **Fonzi, Giorgio**
**Via Isonzo 6 Edificio C/18**
**I-21044 Gerenzano (Varese)(IT)**

(74) Representative: **Porsia, Attilio et al**
**c/o Succ. Ing. Fischetti & Weber-Dr. Porsia**
**Via Caffaro 3/2**
**I-16124 Genova(IT)**

(54) **Method of dry-forming sheet products containing super-absorbent polymers mixed with cellulosic fibres, and absorbent products thus obtained.**

(57) Method of dry-forming sheet products comprising super-absorbent polymer mixed with cellulose fibres, comprising the steps of : (a) forming a suspension, in an air stream, of an intimate mixture of cellulose fibres and super-absorbent polymer; (b) feeding said suspension to a single or multiple head for dry-forming sheets of paper; (c) laying-down said intimate mixture of cellulose fibres and super-absorbent polymer onto a moving web for dry-forming sheets of paper; (d) binding together the thus laid-down layer of fibres and polymer by means of a resin dispersion, of the type which is used for the dry-formation of paper sheets, and calendering it so as to dry-form a sheet constituted by a mixture of super-absorbent polymer and cellulose fibres. The product thus obtained shows characteristics of absorbency, resistance and distributing of the absorbed liquids, which are decidedly better than those of the known composite sheets, such as those comprising one or more supporting layers of tissue paper and/or dry-formed paper having a layer of super-absorbent polymer disposed thereon.

## Method of dry-forming sheet products containing super-absorbent polymers mixed with cellulose fibres, and absorbent products thus obtained

This invention relates to the dry-forming of sheet products containing super-absorbent polymers.

It is known how to form sheets of cellulose fibres by means of a method of dry-forming wherein the cellulose fibres suspended in an air stream are laid down onto one face of a moving permeable forming surface or web, with the aid of a suitable vacuum created on the opposite face of said surface, whereafter the fibrous ribbon-like layer thus obtained is bonded permanently to obtain a so-called "dry-formed" sheet of paper, first by means of a calendering and embossing step and then by spraying a suitable aqueous dispersion of resin onto said fibrous layer.

It is also known how to produce composite sheets comprising a sheet of dry-formed paper or tissue paper on which there is disposed a layer, nearly always in the form of a granular pwder, of a so-called "super-absorbent polymer", thus forming a composite sheet wherein the dry-formed paper sheet serves as a support for the super-absorbent polymer, for manufacturing absorbent products, such as sanitary pads or the like. Quite often, the layer of super-absorbent polymer of these composite sheets is further covered by a second layer of either tissue paper or dry-formed paper in order to avoid a direct contact of the super-absorbent polymer with the skin of a user.

These known manufacturing methods of composite sheets comprising one or more supporting and/or covering layers of either dry-formed paper or tissue paper and a layer of super-absorbent polymer are expensive, in that they require various processing steps; they are difficult to be carried out due to the difficulty for fixing the layer of super-absorbent polymer, which is in the form of granular powder, permanently to the layer, or layers, of either dry-formed paper or tissue paper, whereby the products tend to exfoliate easily; and, moreover, they do not permit a full exploitation of the absorbency of the super-absorbent polymer, due to the tendency of the super-absorbent polymer to cover itself with a colloidal film limiting or hindering the penetration of the water in the innermost portions of the polymer.

By the term "super-absorbent polymers" we mean polymers with a base of high-molecular-weight polyelectrolytes, for example, polyacrylates or "graft" polymers with a base of starch and polyacrylate. Typical of such polymers which are commercially available in the powdered or granular form are those being marked under the name WATERLOCK (R) by Grain Processing, AQUAKEEP (R) by Norsolor, AQUASTORE (R) by Cyanamid, and SANWET (R) by San. Typical of such polymers which are commercially available in form of fibrils or of extra-short fibres, are those produced by DOW or by STEEN containing polyethylenes and polypropylenes grafted with polyacrylates. The above mentioned super-absorbent polymers have very high water absorbencies, of the order of 500 to 1,000 parts of water (by weight) per 1 part of polymer, and high absorbencies of saline solutions (Nacl 1%), of the order of 20 to 100 parts (by weight) of solution per 1 part of polymer.

The present invention, therefore, relates to a method of dry-forming sheets of cellulose fibres containing a super-absorbent polymer, which overcomes the disadvantages of the known methods.

A further object of the present invention is to provide a new dry-formed product having a base of cellulose fibres and super-absorbent polymers, obtained by the method set forth above.

According to the present invention, we have found out that a sheet product having very high absorbency may be obtained by: (a) forming a suspension, in an air stream, of an intimate mixture of cellulose fibres and super-absorbent polymer; (b) feeding said suspension to a head for dry-forming sheets of paper; (c) laying-down said intimate mixture of cellulose fibres and super-absorbent polymer onto a web for dry-forming sheets of paper; (d) binding together the thus laid-down layer of fibres and polymer by calendering and embossing it and successively by means of a resin dispersion of the type which is used for the dry-formation of paper sheets.

The product which is obtained by the method set forth above has characteristics of absorbency, resistance and distribution of the absorbed liquids, which are better then those of the known composite sheets, and this is due also to the fact that - since the particles of superabsorbent polymer are distributed uniformly in the supporting body of cellulose fibres - said cellulose fibres exert such a spreading and distributing action of the water on all the particles of polymer, that cannot be obtained by the known products, which are constituted by a layer of polymer and one or more layers of supporting material.

Due to the fact that the particles of polymer are distributed uniformly in the body of cellulose fibres, the need is obviated to provide covering sheets to prevent the polymer from contacting the user's skin.

Moreover, we have found out that in order to bind the layers of cellulose fibres and superabsorbent polymer which have been obtained by means of the method described above, the usual aqueous dispersions of resins may be used, for example, 5-20% aqueous dispersions of ethylene vinyl acetate (EVA) with thermo-reticulant, such as used in the dry-forming of paper, without any perceptible swelling of the super-absorbent polymer in the final product thus obtained.

By using as super-absorbent polymenr component polymers in the form of fibres instead of granules, we have found that a still better binding of the layer formed by cellulosic fibres and polymeric fibres may be obtained, with improved results in the final product obtained.

According to a still further embodiment of the invention, by using a dry-forming plant provided with two or more series-disposed deposition heads, it is possible to obtain a composite product formed by a very thin layer of cellulosic fibres, on which a layer of the mixture of cellulosic fibres and super-absorbent polymer is deposited.

In the manufacturing of the absorbent sheets according to the invention, the mixture of super-absorbent polymer and cellulose fibres which is fed to the dry-forming plant is in a weight percentage ranging from 5 parts of super-absorbent polymer and 95 parts of cellulose fibres to 70 parts of polymer and 30 parts of cellulose fibres.

Advantageously, however, we have found out that optimum results are obtained by using in the mixture a weight ratio of superabsorbent polymer/cellulose fibres of 1-2/3-4.

The amount of resin dispersion which is used to bind together the products of cellulose fibres and super-absorbent polymer according to the invention ranges from 5 to 12 parts (by weight) of dispersion per 100 parts (by weight) of the mixture cellulose fibres/super-absorbent polymer.

Some practical embodiments of the invention are described below by way of non-limiting example.

Example 1

An intimate mixture formed of 70 parts wt% of cellulose fibres and 30 parts wt% of super-absorbent polymer AQUAKEEP (R) from Norsolor, constituted by a polyacrylate-base polymer in powder form, is fed while suspended in an air stream to the forming head of a plant for manufacturing dry-formed paper, for example, to the forming head of the plant which is described in the European Patent Application No. 85104333.1 filed on April 10, 1985 in the name of the same Applicant. Said mixture is laid down onto the web for the dry-forming of paper of said plant, in the form of a layer of cellulose fibres containing, intimately dispersed therein, the particles of super-absorbent polymer. The sheet of fibres/super-absorbent polymer is then submitted to calendering and embossing between heated cylinders, and the thus solidified layer is sprayed uniformly with a 10% aqueous dispersion of EVA containing a thermoreticulant, in a ratio of 8 gr. of dispersion per 100 grams of the mixture fibres/super-absorbent polymer. After drying and thermoreticulation we obtain a sheet of cellulose fibres and super-absorbent polymer having excellent characteristics of cohesion and resistance, and having an absorbency of 3000 to 5000 grams of 1% NaCl solution per 100 grams of product.

This sheet may be used as it is for manufacturing absorbent cloths, absorbent pads, or the like.

Example 2

The same procedure as described in Example 1 is performed, by using as web-forming material an intimate mixture of 60 parts b.w. of cellulose fibres and 40 parts b.w. of a super-absorbent polymer in the form of short fibres, produced by STEEN, and formed by polyethylene grafted with polyacrylates.

Example 3

The same procedures as described in Examples 1 and 2 is performed, in a modified dry forming plant provided with two deposition heads series-mounted, and by feeding the first head with a dispersion of cellulose fibres and the second head, which is disposed down-stream to the first head with respect to the direction of movement of the underlying deposition web, with the mixture of cellulosic fibres and superabsorbent polymer according to the invention.

Claims

1. A method of dry-forming sheet products based on a super-absorbent polymer dispersed in a matrix of cellulose fibres, characterized by the following steps: (a) forming a suspension, in an airstream, of an intimate mixture of cellulose fibres and a super-absorbent polymer; (b) feeding said suspension to a forming head of a plant for manufacturing dry-formed sheets of paper; (c) laying down said intimate mixture of cellulose fibres and super-absorbent polymer onto a web for dry-forming paper sheets in said plant; and (d) binding together the thus laid-down layer of fibres and polymer, firstly by hot calendering and embossing steps and then by means of a resin dispersion of the type which is used for the dry-formation of paper sheets, so as to obtain a sheet comprising a matrix of cellulose fibres containing a dispersion of particles of super-absorbent polymer.

2. A method according to claim 1 chracaterized by the fact that the said plant for manufacturing dry formed sheets is provided with at least two formation heads, the first one of which is supplied with a suspension of cellulosic fibres in air, and the second one of which, which is disposed downstream to the first one in the direction of movement of the said formation web, is supplied with a suspension formed by the said mixture of cellulosic fibres and super-absorbent polymer in air.

3. A method according to claims 1 or 2, characterized in that said mixture of cellulose fibres and super-absorbent polymer contains, as expressed in parts by weight, 5 parts of polymer and 95 parts of cellulose fibres to 70 parts of polymer and 30 parts of cellulose fibres.

4. A method according to claim 3, wherein said weight ratio of super-absorbent polymer/cellulose fibres in said mixture is, preferably, in the range of 1-2 / 3-4.

5. A method according to the preceding claims, wherein said super-absorbent polymers are polyelectrolytes grafted on starch, or polyacrylate-based polymers, or hydrolysis products from polyacrylonitrile grafted on starch and hydrolyzed.

6. A method according to claims 1 to 3, characetrized by the fact that said super-absorbent polymers are polyacrylates grafted on polyethylene and/or polypropylene, in form of fibres having dimensions like to that of a cellulosic pulp used in the production of the dry-formed paper.

7. A method according to claims 1 to 3, characterized by the fact that the said superabsosrbent polymers are polyacrilates grafted on polyethylene or polyacrylonitriles, in form of short fibres having e length in the range between 2 and 6 mm.

8. A method according to the preceding claims, wherein the sheets are bonded by means of a 5-20% aqueous dispersion of ethylene-vinylacetate, preferably in the ratio of 5-12 grams of dispersion per 100 grams of product to be bound.

9. A method of dry-forming sheet products based on a super-absorbent polymer dispersed in a matrix of cellulose fibres, substantially as described and as claimed in the preceding claims.

10. An industrial product comprising a dry-formed sheet based on a super-absorbent polymer dispersed in a matrix of cellulose fibres obtained by means of the method according to the preceding claims, containing 5 to 70 wt% of super-absorbent polymer and 95 to 30 wt% of cellulose fibres.

11. The use of the product according to claim 10 for manufacturing absorbent cloths, pads, bed-sheets, wiping cloths and other similar absorbent articles, which are intended to absorb and retain great percentages of liquids.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 122 042 (THE PROCTER & GAMBLE CO.) <br> * Claims 1-7,9-12; page 7; page 9, lines 21-36; page 11, last paragraph; examples I,III-IX * | 1-5,9- 11 | D 21 H 5/26 <br> A 61 F 13/18 <br> A 47 L 13/16 |
| A | US-A-4 360 022 (A. USAMI et al.) <br> * Figures 1-3; column 1; column 2, lines 1-40 * | 1,5,9, 11 | |
| A | US-A-4 160 059 (T. SAMEJIMA) <br><br> * Column 2, lines 44-67; column 3, line 15 - column 4, line 54 * | 1,3,5, 9-11 | |
| A | GB-A-1 313 134 (THE ASSOCIATED PAPER MILLS) <br> * Whole document * | 1,8,9, 11 | **TECHNICAL FIELDS SEARCHED (Int. Cl.4)** <br><br> A 61 F <br> D 04 H <br> D 21 H |

-----

The present search report has been drawn up for all claims

| Place of search | Date of completion of the search | Examiner |
|---|---|---|
| THE HAGUE | 02-11-1987 | NESTBY K. |